# FASCICULE DE BREVET EUROPEEN

(11) **EP 2 120 774 B1**
(45) Date de publication et mention de la délivrance du brevet: **22.09.2010**
(21) Numéro de dépôt: 08706369.9
(22) Date de dépôt: 19.02.2008
(51) Int. Cl.: A61C 1/08, A61B 17/16, C22C 45/00, C22C 45/10

(54) **PIECE A MAIN A USAGE DENTAIRE OU CHIRURGICAL**
TRAGBARES INSTRUMENT FÜR DIE DENTALE UND CHIRURGISCHE VERWENDUNG
HANDHELD PART FOR DENTAL OR SURGICAL USE

(30) Priorité: 16.03.2007 CH 4502007
(43) Date de publication de la demande: 25.11.2009
(73) Titulaire: Bien-Air Holding SA, 2500 Bienne 6 (CH)
(72) Inventeur: MOSIMANN, Vincent, CH-2520 La Neuveville (CH)
(74) Mandataire: Ravenel, Thierry Gérard Louis
(86) Numéro de dépôt international: PCT/CH2008/000069
(87) Numéro de publication internationale: WO 2008/113192

(56) Documents cités:
- WO-A2-2004/021913
- DE-A1-102007 030 019
- US-A1- 2004 267 349
- US-A1- 2005 191 597

## Description

La présente invention concerne une pièce à main à usage dentaire ou chirurgical. Plus précisément, la présente invention concerne un tel instrument à usage dentaire ou chirurgical dont le corps est réalisé dans un matériau léger et facile à usiner.

Il existe principalement deux familles d'instruments à main à usage dentaire ou chirurgical. La première de ces familles regroupe ce que l'on appelle les turbines, à savoir des instruments dont l'outil, par exemple une fraise, est couplé à un moteur entraîné par de l'air sous pression. Un tel moteur peut tourner à plusieurs centaines de milliers de tours par minute avec une pression d'air typiquement de l'ordre de 2,5 à 3 bars. Une telle turbine comprend un corps qui permet au praticien de tenir son instrument en main. Ce corps est classiquement relié à une unité d'alimentation électrique et de fourniture de fluides (air, eau) via un raccord et un tuyau souple.

La seconde famille d'instruments à usage dentaire ou chirurgical regroupe ce que l'on appelle les pièces à main et les contre-angles. Les pièces à main et les contre-angles diffèrent pour l'essentiel en ce que le corps des pièces à main est sensiblement droit tandis que celui des contre-angles forme un angle en un endroit de sa longueur. Pour le reste, ces deux types d'instruments se ressemblent beaucoup. Un moteur, fixé sur la pièce à main ou le contre-angle et capable de tourner à plusieurs dizaines de milliers de tours par minute, est relié via un tuyau à raccord fixe ou tournant à l'unité d'alimentation.

Pour des raisons de commodité, on désignera dans ce qui suit les instruments à usage dentaire ou chirurgical tels que décrits ci-dessus par l'expression générique pièce à main, sachant que cette expression recouvre tout aussi bien les pièces à main proprement dites que les turbines et les contre-angles.

Les pièces à main se composent essentiellement d'un corps qui permet de les tenir en main et d'une tête qui porte notamment l'outil. Ces éléments sont classiquement fabriqués à partir d'un barreau de laiton ou d'acier inoxydable. Ils présentent des formes aussi bien extérieures qu'intérieures extrêmement complexes dont l'usinage (débitage, perçage, filetage, matriçage etc.) est long et coûteux et nécessite des machines sophistiquées qui peuvent comprendre jusqu'à huit axes d'usinage différents. En outre, quelque soit la sophistication des machines d'usinage utilisées, on est toujours limité par les formes que l'on peut donner à ces éléments.

On notera par ailleurs que les matériaux utilisés, laiton ou acier inoxydable, présentent des avantages mais aussi des inconvénients. Le laiton, par exemple, est un matériau très apprécié des constructeurs car il est facile à usiner. Par contre, le laiton présente l'inconvénient de s'oxyder facilement et doit donc subir des traitements de surface par dépôts de couches de matériaux inoxydables. Ces couches, relativement fragiles, ont tendance à s'user et à se rayer facilement, pouvant laisser apparaître par endroits la couche de laiton sous-jacente, ce qui donne aux praticiens le sentiment d'un instrument de qualité médiocre. En outre, certaines des couches contiennent du nickel au contact duquel le praticien peut développer des allergies lorsque ces couches sont mises à nu.

Au contraire, l'acier inoxydable est, par définition, un matériau résistant bien à la corrosion et ne nécessitant donc pas de traitement spécifique de ce point de vue. Par ailleurs, l'acier inoxydable est plus léger que le laiton et permet donc de réaliser des pièces à main qui pèsent moins dans la main du praticien, réduisant ainsi la fatigue que celui-ci peut ressentir après plusieurs heures de travail. Mais l'acier inoxydable présente l'inconvénient d'être difficile à usiner.

Le document US 2005 0191597 A1 décrit une pièce à main à usage dentaire ayant un corps en alliage métallique, par exemple en inox, réalisé par moulage par injection métallique.

Pour remédier à ces problèmes, on a déjà réalisé certains composants de pièces à main par injection de matériaux plastiques (éléments pour le raccordement des pièces à main à l'unité d'alimentation et de contrôle, éléments isolants pour connecteurs électriques, ailettes des turbines etc.). Certains des matériaux plastiques utilisés sont biocompatibles, ce qui permet de réaliser des composants destinés à venir au contact du praticien ou du patient. L'injection de matériaux plastiques permet par ailleurs de s'affranchir de presque toutes les contraintes en termes de formes des éléments à réaliser. Elle permet également de réaliser ces éléments avec une grande précision et de les livrer quasi prêts à l'emploi après démoulage. Néanmoins, l'utilisation des matériaux plastiques ne s'est pas généralisée car ces matériaux posent notamment des problèmes de tenue mécanique dans le temps et ne jouissent pas d'une bonne image auprès des praticiens.

Il existait donc, dans l'état de l'art, un besoin pour un matériau permettant de combiner les avantages des matériaux plastiques (légèreté, liberté au niveau des formes, précision, répétitivité et rapidité de l'injection) et des matériaux métalliques (longévité, tenue mécanique).

La présente invention a pour but de répondre à cette attente en procurant une pièce à main à usage dentaire ou chirurgical comprenant des composants fixes et des composants mobiles, **caractérisée en ce que** certains au moins de ces composants fixes et/ou mobiles sont réalisés en un alliage métallique solidifié au moins partiellement en phase amorphe dans le volume.

Les composants fixes d'une pièce à main comprennent notamment la tête et le manche de telles pièces à main. Les composants mobiles comprennent entre autres les roues dentées, les entraîneurs pour transmettre le couple moteur aux outils et certains composants des moteurs électriques.

Le principal obstacle qui, jusqu'à présent, s'opposait à l'emploi de tels alliages métalliques amorphes dans le volume résidait dans le fait que l'on pensait devoir recourir à un outil de production spécifique à de tels matériaux. Or, la demanderesse s'est rendu compte que les matériaux alliés amorphes pouvaient être injectés avec les mêmes machines que celles habituellement utilisées pour l'injection des matériaux plastiques. Ceci est notamment dû au fait que ces alliages présentent typiquement une température de ramollissement et de fusion faible, inférieure ou égale à 600°C. Il n'est donc pas nécessaire de devoir procéder à de lourds investissements pour faire l'acquisition d'un nouvel outil de production. On jouit par ailleurs d'une grande liberté du point de vue des formes qu'il est possible d'impartir aux différents composants de même que d'une grande précision de fabrication. Cette précision de fabrication est notamment due à l'absence de formation de phase cristalline au cours du refroidissement. Le retrait des pièces injectées est donc beaucoup plus faible car on n'observe pas de réarrangement atomique. De plus, les composants sont quasi prêts à l'emploi après démoulage. Seules des étapes de finition telles que surfaçage ou dépôt d'un revêtement décoratif peuvent s'avérer nécessaires. Par ailleurs, les composants métalliques ainsi obtenus présentent une excellente tenue mécanique, sont résistants à la corrosion et sont de 15 à 30% plus légers que les aciers inoxydables en fonction de la composition de l'alliage retenue. En outre, certains de ces alliages sont biocompatibles. On notera également que certains des alliages métalliques amorphes dont il est question ici présentent d'intéressantes propriétés magnétiques, notamment de faibles pertes par courants de Foucault, ce qui les destine tout naturellement à la réalisation de certaines pièces de moteurs électriques.

Selon une caractéristique complémentaire de l'invention, l'alliage métallique comprend au moins 50% en volume de phase amorphe.

Selon une autre caractéristique de l'invention, l'alliage métallique est amorphe dans tout le volume.

Tout alliage métallique qui solidifie en phase amorphe dans tout le volume peut être utilisé dans le cadre de la présente invention. La solidification dans le volume des alliages amorphes se réfère à la famille des alliages amorphes qui peuvent être refroidis à des vitesses de refroidissement aussi faibles que 500° K/sec ou moins, et qui conservent sensiblement leur structure atomique amorphe. Autrement dit, ces alliages métalliques amorphes présentent dans le volume, d'un point de vue cristallographique, une structure amorphe dans laquelle quelques grains monocristallins peuvent être dispersés.

Un exemple typique d'alliages métalliques amorphes qui solidifient convenablement en phase amorphe dans tout le volume est donné par les alliages Zr-Al-R, Zr-Al-Ni-Cu et Zr-Ti-Ni-Cu-Be auxquels peut être ajouté de l'yttrium. Plus précisément, ces alliages métalliques sont décrits par les formules moléculaires suivantes :
- ZrₓAl_{y}R_{z} avec x, y < 100 et z = x-y;
- (Zr₅₅ Al₁₅ Ni₁₀ Cu₂₀) ₁₀₀₋ₓ Yₓ avec x compris entre 0 et 10;
- (Zr₆₅ Al_{7.5} Ni₁₀ Be_{22.5}) ₁₀₀₋ₓ Yₓ avec x compris entre 0 et 6;
- (Zr₄₁ Ti₁₄ Cu_{12.5} Ni₁₀ Be_{22.5}) ₉₈ Y₂, et
- Zr₃₄ Ti₁₅ Cu₁₂ Ni₁₁ Be₂₈ Y₂.

Il va de soi que la présente invention n'est pas limitée aux modes de réalisation qui viennent d'être décrits et que diverses modifications et variantes simples peuvent être envisagées par l'homme du métier sans sortir du cadre de la présente invention tel que défini par les revendications annexées à la présente demande de brevet. On notera en particulier qu'une des caractéristiques spécifique à ces alliages métalliques amorphes réside dans le fait qu'ils passent de la phase solide à la phase liquide en passant par une nette phase plastique déformable, contrairement aux métaux classiques qui passent directement de la phase solide à la phase liquide. Cette caractéristique rend possible l'utilisation de moyens de mise en oeuvre habituellement employés pour d'autres matériaux.

## Revendications

1. Pièce à main à usage dentaire ou chirurgical comprenant des composants statiques et des composants dynamiques, **caractérisée en ce que** certains au moins de ces composants statiques et/ou dynamiques sont réalisés en un alliage métallique solidifié en phase amorphe dans le volume.

2. Pièce à main selon la revendication 1, **caractérisée en ce que** l'alliage métallique comprend au moins 50% en volume de phase amorphe.

3. Pièce à main selon la revendication 2, **caractérisée en ce que** l'alliage métallique est amorphe dans tout le volume.

4. Pièce à main selon l'une quelconque des revendications 1 à 3, **caractérisée en ce** l'alliage métallique amorphe présente une température de ramollissement et de fusion inférieure ou égale à 600°C.

5. Pièce à main selon l'une quelconque des revendications 1 à 4, **caractérisée en ce que** l'alliage métallique amorphe présente, entre la phase solide et la phase liquide une phase dans laquelle il est déformable plastiquement.

6. Pièce à main selon l'une quelconque des revendications 1 à 5, **caractérisée en ce que** l'alliage métallique amorphe est biocompatible.

7. Pièce à main selon l'une quelconque des revendications 1 à 6, **caractérisée en ce que** l'alliage métallique amorphe est un alliage de Zr-Al-R, de Zr-Al-Ni-Cu ou de Zr-Ti-Ni-Cu-Be.

8. Pièce à main selon la revendication 7, **caractérisée en ce que** l'alliage métallique amorphe de Zr-Al-R, de Zr-Al-Ni-Cu ou de Zr-Ti-Ni-Cu-Be comprend en outre de l'yttrium.

9. Pièce à main selon la revendication 8, **caractérisée en ce que** l'alliage métallique amorphe est décrit par l'une quelconque des formules moléculaires suivantes :
- ZrₓAl_{y}R_{z} avec x, y < 100 et z = x - y;
- (Zr₅₅ Al₁₅ Ni₁₀ Cu₂₀) ₁₀₀₋ₓ Yₓ avec x compris entre 0 et 10;
- (Zr₆₅ Al_{7.5} Ni₁₀ Be_{22.5}) ₁₀₀₋ₓ Yₓ avec x compris entre 0 et 6;
- (Zr₄₁ Ti₁₄ Cu_{12.5} Ni₁₀ Be_{22.5}) ₉₈ Y₂, ou
- Zr₃₄ Ti₁₅ Cu₁₂ Ni₁₁ Be₂₈ Y₂.

## Claims

1. Handpiece for dental or surgical use including static components and dynamic components, **characterized in that** at least some of said static and/or dynamic components are made of a metal alloy bulk solidified in an amorphous phase.

2. Handpiece according to claim 1, **characterized in that** the metal alloy includes at least 50% bulk amorphous phase.

3. Handpiece according to claim 2, **characterized in that** the metal alloy is entirely bulk amorphous.

4. Handpiece according to any of claims 1 to 3, **characterized in that** the amorphous metal alloy has a softening and melting temperature lower than or equal to 600°C.

5. Handpiece according to any of claims 1 to 4, **characterized in that**, between the solid phase and the liquid phase, the amorphous metal alloy has a phase in which it is plastically deformable.

6. Handpiece according to any of claims 1 to 5, **characterized in that** the amorphous metal alloy is biocompatible.

7. Handpiece according to any of claims 1 to 6, **characterized in that** the amorphous metal alloy is an alloy of Zr-Al-R, Zr-Al-Ni-Cu or Zr-Ti-Ni-Cu-Be.

8. Handpiece according to claim 7, **characterized in that** the amorphous metal alloy of Zr-Al-R, Zr-Al-Ni-Cu or Zr-Ti-Ni-Cu-Be also includes yttrium.

9. Handpiece according to claim 8, **characterized in that** the amorphous metal alloy is described by any of the following molecular formulae:
- ZrₓAl_{y}R_{z} with x, y < 100 and z = x-y;
- Zr₅₅ Al₁₅ Ni₁₀ Cu₂₀) ₁₀₀₋ₓ Yₓ with x comprised within 0 and 10;
- Zr₆₅ Al_{7.5} Ni₁₀ Be_{22.5}) ₁₀₀₋ₓ Yₓ with x comprised within 0 and 6;
- Zr₄₁ Ti₁₄ Cu_{12.5} Ni₁₀ Be_{22.5}) ₉₈ Y₂ and ;
- Zr₃₄ Ti₁₅ Cu₁₂ Ni₁₁ Be₂₈ Y₂.

## Patentansprüche

1. Handteil für zahnärztliche oder chirurgische Verwendung, das statische Komponenten und dynamische Komponenten enthält, **dadurch gekennzeichnet, dass** wenigstens Bestimmte dieser statischen und/oder dynamischen Komponenten aus einer Metalllegierung verwirklicht sind, die in der amorphen Phase im Volumen verfestigt ist.

2. Handteil nach Anspruch 1, **dadurch gekennzeichnet, dass** die Metalllegierung die amorphe Phase in einem Anteil von wenigstens 50 Vol.-% enthält.

3. Handteil nach Anspruch 2, **dadurch gekennzeichnet, dass** die Metalllegierung im gesamten Volumen amorph ist.

4. Handteil nach einem der Ansprüche 1 bis 3, **dadurch gekennzeichnet, dass** die amorphe Metalllegierung eine Erweichungs- und Schmelztemperatur kleiner oder gleich 600 °C aufweist.

5. Handteil nach einem der Ansprüche 1 bis 4, **dadurch gekennzeichnet, dass** die amorphe Metalllegierung zwischen der festen Phase und der flüssigen Phase eine Phase aufweist, in der sie plastisch verformbar ist.

6. Handteil nach einem der Ansprüche 1 bis 5, **dadurch gekennzeichnet, dass** die amorphe Metalllegierurig biokompatibel ist.

7. Handteil nach einem der Ansprüche 1 bis 6, **dadurch gekennzeichnet, dass** die amorphe Metalllegierung eine Legierung aus Zr-Al-R, aus Zr-Al-Ni-Cu oder aus Zr-Ti-Ni-Cu-Be ist.

8. Handteil nach Anspruch 7, **dadurch gekennzeichnet, dass** die amorphe Metalllegierung aus Zr-Al-R, aus Zr-Al-Ni-Cu oder aus Zr-Ti-Ni-Cu-Be außerdem Yttrium enthält.

9. Handteil nach Anspruch 8, **dadurch gekennzeichnet, dass** die amorphe Metalllegierung durch eine der folgenden molekularen Formeln beschrieben wird:
- ZrₓAl_{y}R_{z}, mit x, y < 100 und z = x - y;
- (Zr₅₅Al₁₅Ni₁₀Cu₂₀)₁₀₀₋ₓYₓ, mit x im Bereich von 0 bis 10;
- (Zr₆₅Al_{7.5}Ni₁₀Be_{22.5})₁₀₀₋ₓYₓ, mit x im Bereich von 0 bis 6;
- (Zr₄₁Ti₁₄Cu_{12.5}Ni₁₀Be_{22.5})₉₈ Y₂; oder
- (Zr₃₄Ti₁₅Cu₁₂Ni₁₁Be₂₈Y₂).
